# EUROPÄISCHE PATENTANMELDUNG

(11) **EP 1 419 736 A1**
(43) Veröffentlichungstag der Anmeldung: **19.05.2004**
(21) Anmeldenummer: 03022996.7
(22) Anmeldetag: 10.10.2003
(51) Int. Cl.: A61B 6/04, A61G 13/10

(54) **Plattenförmige Vorrichtung zur Lagerung eines Körperteiles eines Patienten**

(30) Priorität: 12.11.2002 DE 10253877
(71) Anmelder: TRUMPF Medizin Systeme GmbH., 07318 Saalfeld (DE)
(72) Erfinder: Ludwig, Carmen, 07551 Gera (DE); Stolze, Dirk, 07318 Saalfeld (DE)
(74) Vertreter: Karrais, Martin

(57) **Zusammenfassung**

Die Erfindung betrifft eine plattenförmige Vorrichtung zur Lagerung eines Körperteiles eines Patienten, wobei die Vorrichtung eine Lagerfläche ausbildet und eine mehrteilige Tragstruktur aufweist mit einem Hauptteil und mindestens einem Zusatzteil, das mittels mindestens eines Koppelelementes mit dem Hauptteil lösbar verbindbar ist. Um die plattenförmige Vorrichtung derart weiterzubilden, daß einerseits eine stabile Lagerung des Patienten sichergestellt ist und andererseits eine Durchleuchtung des Patienten unter einem möglichst großen Winkel schräg zur Körperlängsachse des Patienten ermöglicht wird, wird erfindungsgemäß vorgeschlagen, daß das Hauptteil, das Zusatzteil und das Koppelelement aus einem eine hohe Transparenz für Röntgenstrahlen aufweisenden Material gefertigt sind und daß das Zusatzteil bezogen auf die Körperlängsachse des Patienten seitlich am Hauptteil abnehmbar gehalten ist.

## Beschreibung

Die Erfindung betrifft eine plattenförmige Vorrichtung zur Lagerung eines Körperteiles eines Patienten, wobei die Vorrichtung eine Lagerfläche ausbildet und eine mehrteilige Tragstruktur aufweist mit einem Hauptteil und mindestens einem Zusatzteil, das mittels mindestens eines Koppelelementes mit dem Hauptteil lösbar verbindbar ist.

Derartige Vorrichtungen bilden zumindest einen Teil einer Patientenliege oder eines Operationstisches und kommen in vielfältiger Form zum Einsatz. Mit ihrer Hilfe kann ein Patient auf der Lagerfläche zuverlässig gelagert werden für therapeutische Behandlungen und chirurgische Eingriffe und auch für diagnostische Verfahren, insbesondere für Untersuchungen mittels eines Computer-Tomographen (CT) oder mittels Magnet-Resonanz-Tomographie (MRT).

Um eine stabile Lagerung des Patienten sicherstellen zu können, ist es wünschenswert, wenn die Lagerfläche eine gewisse Mindestbreite aufweist. Andererseits muß jedoch bei einer Durchleuchtung des Patienten in einem Computer-Tomographen die Lagerfläche zwischen das bildgebende System des Tomographen, also zwischen der Röntgenröhre und die dieser gegenüberliegenden Sensoren, eingeführt werden. Hierbei stellt sich bei einer Durchleuchtung des Patienten in einem Winkel schräg zu dessen Körperlängsachse das Problem, daß der maximale Winkel, unter dem eine Durchleuchtung schräg zur Patientenlängsachse vorgenommen werden kann, durch die Breite der Lagerfläche beschränkt ist.

Aufgabe der vorliegenden Erfindung ist es, eine plattenförmige Vorrichtung der eingangs genannten Art derart weiterzubilden, daß sie einerseits eine stabile Patientenlagerung sicherstellt und andererseits eine Durchleuchtung des Patienten in einem möglichst großen Winkel schräg zur Körperlängsachse des Patienten ermöglicht.

Diese Aufgabe wird bei einer plattenförmigen Vorrichtung der gattungsgemäßen Art erfindungsgemäß dadurch gelöst, daß das Hauptteil, das mindestens eine Zusatzteil und das mindestens eine Kopplungselement aus einem eine hohe Transparenz für Röntgenstrahlen aufweisenden Material gefertigt sind, und daß das Zusatzteil zur Verschmälerung der Lagerfläche bezogen auf die Körperlängsachse des Patienten seitlich am Hauptteil abnehmbar gehalten ist.

Eine derartige Ausgestaltung hat den Vorteil, daß die gesamte Tragstruktur der Vorrichtung röntgenstrahlendurchlässig ausgestaltet werden kann, indem sowohl das Hauptteil als auch das Zusatzteil und das mindestens eine Koppelelement aus einem eine hohe Transparenz für Röntgenstrahlung aufweisenden Material gefertigt sind. Dies ermöglicht es insbesondere, auch im Bereich der Koppelelemente Computer-Tomographie-Aufnahmen mit hoher Qualität zu erzeugen. Soll eine CT-Aufnahme senkrecht zur Körperlängsachse des Patienten angefertigt werden, so kann das Zusatzteil mit dem Hauptteil verbunden werden und dadurch die Tragstruktur eine breite Lagerfläche für den Patienten ausbilden. Soll der Patient jedoch beispielsweise im Schulter- oder Hüftbereich schräg zu dessen Körperlängsachse durchleuchtet werden, indem die zum Einsatz kommende Röntgenröhre sowie die dieser gegenüberliegenden Sensoren des Computer-Tomographen unter Aufrechterhaltung eines gleichbleibenden Abstandes zwischen Röntgenröhre und Sensoren schräg zur Körperlängsachse ausgerichtet werden, so läßt sich die Lagerfläche auf einfache Weise verschmälern, indem das Zusatzteil der Tragstruktur vom Hauptteil abgenommen wird. Eine Behinderung des Computer-Tomographen bei einer Durchleuchtung des Patienten schräg zu dessen Körperlängsachse durch die Tragstruktur wird daher zuverlässig vermieden.

Von Vorteil ist es, wenn das Hauptteil und/oder das mindestens eine Zusatzteil plattenförmig ausgestaltet sind. Dies ermöglicht eine kostengünstige Herstellbarkeit der Vorrichtung, wobei sichergestellt ist, daß die Tragstruktur das Gewicht des Patienten aufnimmt, ohne daß zusätzliche Stützelemente zum Einsatz kommen müssen.

Bei einer besonders bevorzugten Ausgestaltung der erfindungsgemäßen plattenförmigen Vorrichtung umfaßt die Tragstruktur zwei Zusatzteile, die an einander gegenüberliegenden Seiten des Hauptteiles abnehmbar gehalten sind. Die Tragstruktur ist somit insgesamt 3-teilig ausgebildet, wobei zumindest entlang eines Teilbereiches der Tragstruktur drei Tragstrukturteile, nämlich das Hauptteil sowie die beiden Zusatzteile, seitlich nebeneinander angeordnet sind. Je nachdem auf welcher Seite, bezogen auf die Körperlängsachse des Patienten, die Lagerfläche verschmälert werden soll, kann das linke oder das rechte Zusatzteil vom Hauptteil abgenommen werden. Selbstverständlich können auch beide Zusatzteile gleichzeitig vom Hauptteil abgenommen werden, um auf diese Weise eine besonders schmale Lagerfläche erzielen zu können.

Vorzugsweise sind die beiden Zusatzteile spiegelbildlich zueinander ausgestaltet.

Als besonders günstig hat es sich erwiesen, wenn sich das Hauptteil über die gesamte Länge der Tragstruktur erstreckt. Das Hauptteil bildet damit einen Mittelabschnitt der Tragstruktur, der besonders massiv ausgestaltet sein kann und das volle Patientengewicht aufnehmen kann. Ergänzend können dann die Zusatzteile zum Einsatz kommen, die sich zumindest über einen Teilbereich der Gesamtlänge der Tragstruktur erstrecken und wahlweise genutzt werden können, oder aber zur besseren Zugänglichkeit des bildgebenden Systems des Computer-Tomographen bei einer Durchleuchtung des Patienten entfernt werden können.

Vorzugsweise sind an das Hauptteil weitere Tragelemente ankoppelbar, beispielsweise zur Lagerung der Extremitäten oder des Kopfes des Patienten.

Bei einer besonders bevorzugten Ausgestaltung ist das Hauptteil T- oder Y-förmig ausgestaltet. Bei einer derartigen Ausführungsform verschmälert sich das Hauptteil in Richtung der Körperlängsachse des Patienten. Es hat sich gezeigt, daß dadurch die mechanische Belastbarkeit der Tragstruktur verbessert werden kann.

Um der Tragstruktur eine besonders gute Durchlässigkeit für Röntgenstrahlen zu verleihen, ist es günstig, wenn das Hauptteil und/oder das mindestens eine Zusatzteil aus einem Carbonfasermaterial gefertigt sind. Derartige Materialien zeichnen sich durch eine sehr hohe Transparenz für Röntgenstrahlen aus. Sie haben außerdem den Vorteil, daß sie nur ein geringes spezifisches Gewicht aufweisen. Sie eignen sich deshalb besonders zur Ausgestaltung der abnehmbaren Zusatzteile, da deren Handhabung bei einem geringen Gewicht besonders vereinfacht ist. Unter Carbonfasermaterial werden hierbei kohlefaserverstärkte Kunststoffe verstanden.

Günstig ist es, wenn das Hauptteil und/oder das mindestens eine Zusatzteil als massive Platten ausgebildet sind. Das Hauptteil und/oder das Zusatzteil erhalten dadurch eine besonders hohe mechanische Belastbarkeit und können kostengünstig hergestellt werden. Besonders günstig ist es, wenn das Hauptteil und das mindestens eine Zusatzteil aus massivem Carbonfasermaterial hergestellt sind.

Vorzugsweise untergreift das Zusatzteil das Hauptteil. Es hat sich gezeigt, daß dadurch die Belastbarkeit der Tragstruktur im Grenzbereich zwischen dem Hauptteil und einem Zusatzteil verbessert werden kann. So kann beispielsweise vorgesehen sein, daß das Hauptteil und das mindestens eine Zusatzteil in ihrem jeweiligen Anlagebereich einander zugewandte, schräg zur Vertikalen ausgerichtete Seitenflächen aufweisen.

Bei einer besonders bevorzugten Ausführungsform sind das Hauptteil und/oder das mindestens eine Zusatzteil zumindest bereichsweise im Querschnitt trapezförmig ausgestaltet. Bei Einsatz von zwei jeweils an einer Längsseite des Hauptteiles anliegenden Zusatzteilen hat es sich als günstig erwiesen, wenn das Hauptteil in seinem zwischen den beiden Zusatzteilen angeordneten Bereich einen Querschnitt in Form eines symmetrischen Trapezes aufweist.

Wie bereits erläutert, ist erfindungsgemäß vorgesehen, daß nicht nur das Hauptteil sowie das mindestens eine Zusatzteil sondern auch das mindestens eine Koppelelement, mit dessen Hilfe das Zusatzteil lösbar verbindbar am Hauptteil gehalten ist, aus einem eine hohe Transparenz für Röntgenstrahlung aufweisenden Material gefertigt ist. Vorzugsweise ist das mindestens eine Koppelelement aus einem Carbonfasermaterial gefertigt.

Eine besonders einfache Handhabung der erfindungsgemäßen Vorrichtung beim Abnehmen des Zusatzteiles sowie beim Verbinden des Zusatzteiles mit dem Hauptteil kann dadurch erzielt werden, daß das mindestens eine Koppelelement ein bewegbar am Hauptteil oder am Zusatzteil gelagertes Verbindungselement aufweist, das in eine Aufnahme des Zusatzteiles bzw. des Hauptteiles einführbar ist. Mittels des Verbindungselementes kann eine mechanisch belastbare Verbindung zwischen dem Hauptteil und dem Zusatzteil hergestellt werden. Über das Verbindungselement läßt sich die vom Patienten auf das Zusatzteil ausgeübte Gewichtskraft auf das Hauptteil übertragen.

Das Verbindungselement kann beispielsweise in Form eines Querträgers ausgestaltet sein, der quer zur Körperlängsachse des Patienten verschiebbar gelagert ist.

Eine besonders einfache Handhabung beim Abnehmen des Zusatzteiles vom Hauptteil kann dadurch erzielt werden, daß das Verbindungselement in einer am Zusatzteil festgelegten Führung verschiebbar gelagert ist und in eine Aufnahme des Hauptteiles einführbar ist. Zum Abnehmen ist es dann lediglich erforderlich, das Verbindungselement aus der Aufnahme des Hauptteiles herauszuführen, so daß anschließend das Zusatzteil vom Hauptteil abgenommen werden kann. Umgekehrt ist es beim Ankoppeln des Zusatzteiles an das Hauptteil lediglich erforderlich, das am Zusatzteil gelagerte Verbindungselement in die Aufnahme des Hauptteiles einzuführen.

Vorzugsweise ist das Verbindungselement in der Aufnahme verriegelbar, dadurch läßt sich ein unbeabsichtigtes Abtrennen des Zusatzteiles vom Hauptteil zuverlässig vermeiden.

Die erfindungsgemäße plattenförmige Vorrichtung kann die Tischplatte eines Patientenlagerungstisches ausbilden. Es kann allerdings auch vorgesehen sein, daß die plattenförmige Vorrichtung an ein Tischplattensegment eines Patientenlagerungstisches ankoppelbar ist, beispielsweise an ein zentrales Tischplattensegment einer Patientenliege oder eines Operationstisches.

Die nachfolgende Beschreibung einer bevorzugten Ausführungsform der Erfindung dient im Zusammenhang mit der Zeichnung der näheren Erläuterung. Es zeigen:
- Figur 1:: eine schematische Seitenansicht eines Operationstisches mit einer erfindungsgemäßen plattenförmigen Vorrichtung zur Lagerung eines Körperteiles eines Patienten;
- Figur 2:: eine schematische Draufsicht auf den in Figur 1 dargestellten Operationstisch mit einem von einem Hauptteil der plattenförmigen Vorrichtung abgelösten Zusatzteil; und
- Figur 3:: eine Explosionsdarstellung der erfindungsgemäßen plattenförmigen Vorrichtung.

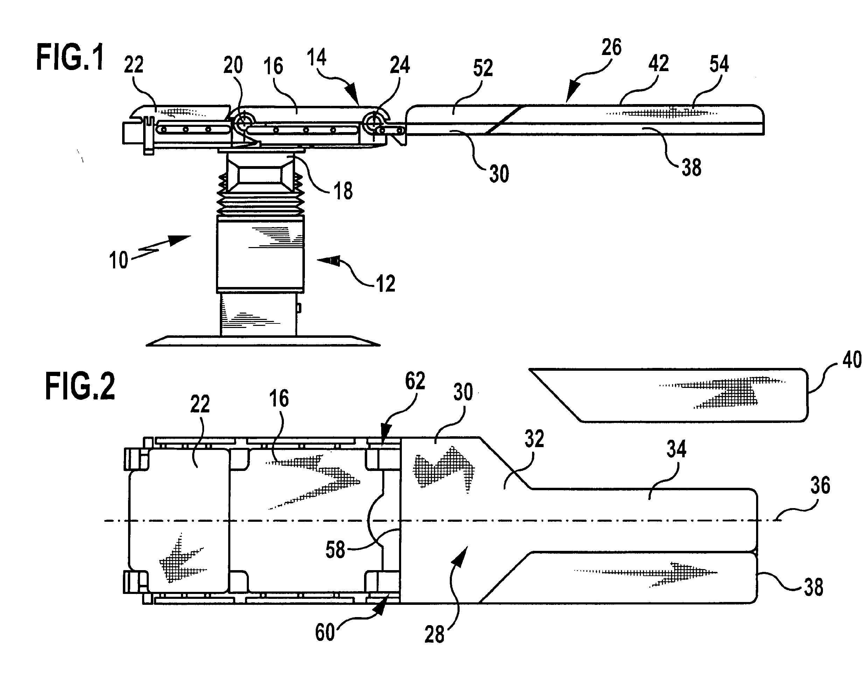

In Figur 1 ist schematisch ein insgesamt mit dem Bezugszeichen 10 belegter Operationstisch dargestellt mit einer Tragsäule 12 und einer mit der Tragsäule 12 lösbar verbindbaren Tischplatte 14. Letztere ist mehrteilig ausgestaltet und umfaßt ein Basissegment 16, das mit einem Tragsäulenkopf 18 der Tragsäule 12 lösbar verbindbar ist und an dem einerseits um eine horizontale Verschwenkachse 20 verschwenkbar ein erstes Endsegment 22 und andererseits um eine ebenfalls horizontal ausgerichtete Verschwenkachse 24 ein zweites Endsegment 26 verschwenkbar gelagert sind. Das zweite Endsegment 26 wird hierbei von einer erfindungsgemäßen plattenförmigen Vorrichtung zur Lagerung eines Körperteiles eines Patienten ausgebildet, dessen Kopf- und Rumpfbereich beispielsweise vom zweiten Endsegment 26 und dessen Bein- und Fußbereich vom Basissegment 16 sowie dem ersten Endsegment 22 abgestützt werden kann. Selbstverständlich kann auch eine umgekehrte Lagerung des Patienten vorgesehen sein dergestalt, daß der Kopf- und Rumpfbereich des Patienten vom ersten Endsegment 22 bzw. dem Basissegment 16 abgestützt wird, während der Bein- und Fußbereich des Patienten vom zweiten Endsegment 26 abgestützt wird.

Die erfindungsgemäße plattenförmige Vorrichtung in Form des zweiten Endsegmentes 26 definiert auf ihrer Oberseite eine Lagerfläche 42 für einen Patienten. Das zweite Endsegment 26 umfaßt ein sich über die gesamte Länge des zweiten Endsegmentes 26 erstreckendes Hauptteil 28, das im wesentlichen Y-förmig ausgestaltet ist und einen sich über die gesamte Breite der Tischplatte 14 erstreckenden Querabschnitt 30 aufweist, an den sich über einen sich in Querrichtung verschmälernden Zwischenabschnitt 32 ein Längsabschnitt 34 anschließt. Das zweite Endsegment 26 weist außerdem zwei spiegelsymmetrisch zur Körperlängsachse 36 eines auf der Tischplatte 14 zu lagernden Patienten ausgestaltete Zusatzteile 38 und 40 auf, die im Bereich des Zwischenabschnittes 32 und des Längsabschnittes 34 seitlich an das Hauptteil 28 angrenzen und mit diesem lösbar verbunden sind. Hierzu sind an den der Lagerfläche 42 abgewandten Unterseiten der Zusatzteile 38 und 40 jeweils zwei Führungshülsen 44 festgelegt, die in Höhe des Übergangsbereiches zwischen dem Zwischenabschnitt 32 und dem Längsabschnitt 34 sowie in Höhe des freien Endes des Längsabschnittes 34 angeordnet sind und jeweils ein Verbindungselement in Form eines quer zur Körperlängsachse 36 verschiebbaren Bolzens 46 aufnehmen, dem an der dem jeweiligen Zusatzteil 38 bzw. 40 zugewandten Seitenkante 48 des Längsabschnittes 34 des Hauptteiles 28 eine Aufnahme 50 zugeordnet ist, in die er eingeführt werden kann zur Herstellung einer lösbaren Verbindung zwischen dem Zusatzteil 38 bzw. 40 einerseits und dem Hauptteil 28 andererseits. Auf ihrer Oberseite tragen das Hauptteil 28 sowie die beiden Zusatzteile 38 und 40 jeweils ein Polstersegment 52, 54 bzw. 56.

Zur Herstellung einer lösbaren Verbindung zwischen dem zweiten Endsegment 26 und dem Basissegment 16 der Tischplatte 14 sind an der dem Längsabschnitt 24 abgewandten Stirnseite 58 des Querabschnittes 30 zwei an sich bekannte und deshalb in der Zeichnung nur schematisch dargestellte Kopplungsteile 60, 32 festgelegt.

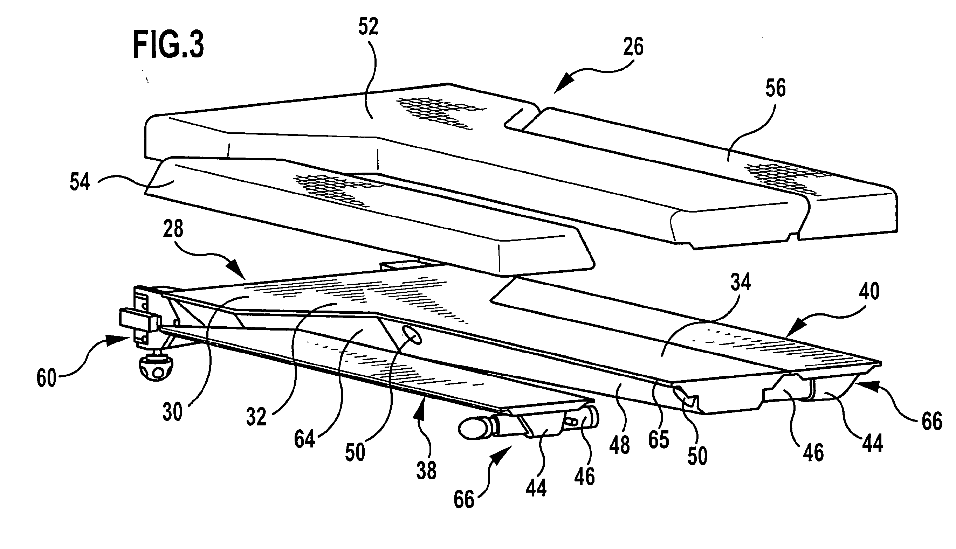

Das Hauptteil 28 sowie die beiden Zusatzteile 38 und 40 sind aus einem Carbonfasermaterial in Form massiv ausgestalteter Platten ausgebildet. Sie nehmen das Gewicht des auf ihnen gelagerten Körperteils des Patienten auf, ohne daß eine zusätzliche Stützstruktur zum Einsatz kommen muß. Im Querschnitt sind die beiden Zusatzteile 38 und 40 ebenso wie der Längsabschnitt 34 des Hauptteils 28 trapezförmig ausgestaltet, dies wird aus Figur 3 deutlich. Der Längsabschnitt 34 weist hierbei eine Querschnittsform in Form eines symmetrischen Trapezes auf, wobei die jeweils einem Zusatzteil 38 bzw. 40 zugewandte Seitenkante 48 des Längsabschnittes 34 ebenso wie die sich daran anschließende Seitenkante 64 des Zwischenabschnittes 32 schräg zur Vertikale nach unten geneigt sind, und die Zusatzteile 38 und 40 untergreifen eine über die Seitenkanten 48 und 64 überstehende Lippe 65 des Hauptteiles 28 im Bereich von dessen Zwischenabschnitt 32 sowie dessen Längsabschnitt 34.

Auch die Führungshülsen 44 sowie die Bolzen 46, die in ihrer Kombination jeweils ein Koppelelement 66 ausbilden zur Herstellung einer lösbaren Verbindung zwischen einem Zusatzteil bzw. 40 und dem Hauptteil 28, sind aus einem Carbonfasermaterial hergestellt, so daß das zweite Endsegment 26 mit Ausnahme der beiden Kopplungsteile 60 und 62 eine sehr hohe Transparenz für Röntgenstrahlung aufweist. Um dies sicherzustellen, sind auch die Polstersegmente 52, 54 und 56 aus einem röntgenstrahlungsdurchlässigen Material gefertigt.

Die 3-teilige Ausgestaltung des zweiten Endsegments 26 in Form des Hauptteiles 28 und der beiden Zusatzteile 38 und 40 ermöglicht es, auf einfache Weise die Lagerfläche 42 des zweiten Endsegments 26 zu verschmälern, indem zumindest ein Zusatzteil 38 oder 40 vom Hauptteil 28 abgenommen wird. Dies ist schematisch in Figur 2 dargestellt. Dadurch wird im seitlichen Randbereich des Hauptteiles 28 in Höhe von dessen Längsabschnitt 34 ein Freiraum geschaffen, in dem zur Durchleuchtung eines Patienten im Winkel bis zu 45° zu dessen Körperlängsachse eine Röntgenröhre oder die diesem zugeordneten Detektoren positioniert werden können. Durch die quer zur Körperlängsachse 36 3-teilige Ausgestaltung des zweiten Endsegmentes 26 kann folglich die Einsatzfähigkeit der Tischplatte 14 bei der Durchleuchtung eines Patienten erheblich gesteigert werden.

## Patentansprüche

1. Plattenförmige Vorrichtung zur Lagerung eines Körperteiles eines Patienten, wobei die Vorrichtung eine Lagerfläche ausbildet und eine mehrteilige Tragstruktur aufweist mit einem Hauptteil und mindestens einem Zusatzteil, das mittels eines Koppelelementes mit dem Hauptteil lösbar verbindbar ist, **dadurch gekennzeichnet, daß** das Hauptteil (28), das mindestens eine Zusatzteil (38, 40) und das mindestens eine Koppelelement (66) aus einem eine hohe Transparenz für Röntgenstrahlen aufweisenden Material gefertigt sind und daß das Zusatzteil (38, 40) zur Verschmälerung der Lagerfläche (42) bezogen auf die Körperlängsachse (36) des Patienten seitlich am Hauptteil (28) abnehmbar gehalten ist.

2. Vorrichtung nach Anspruch 1, **dadurch gekennzeichnet, daß** das Hauptteil (28) und/oder das mindestens eine Zusatzteil (38, 40) plattenförmig ausgestaltet sind.

3. Vorrichtung nach Anspruch 1 oder 2, **dadurch gekennzeichnet, daß** die Tragstruktur zwei Zusatzteile (38, 40) aufweist, die an einander gegenüberliegenden Seiten des Hauptteiles (28) abnehmbar gehalten sind.

4. Vorrichtung nach Anspruch 3, **dadurch gekennzeichnet, daß** die beiden Zusatzteile (38, 40) spiegelbildlich zueinander ausgestaltet sind.

5. Vorrichtung nach einem der voranstehenden Ansprüche, **dadurch gekennzeichnet, daß** sich das Hauptteil (28) über die gesamte Länge der Tragstruktur erstreckt.

6. Vorrichtung nach einem der voranstehenden Ansprüche, **dadurch gekennzeichnet, daß** das Hauptteil (28) T- oder Y-förmig ausgestaltet ist.

7. Vorrichtung nach einem der voranstehenden Ansprüche, **dadurch gekennzeichnet, daß** das Hauptteil (28) und/oder das mindestens eine Zusatzteil (38, 40) aus einem Carbonfasermaterial gefertigt sind.

8. Vorrichtung nach Anspruch 7, **dadurch gekennzeichnet, daß** das Hauptteil (28) und/oder das mindestens eine Zusatzteil (38, 40) als massive Platten ausgebildet sind.

9. Vorrichtung nach Anspruch 8, **dadurch gekennzeichnet, daß** das Hauptteil (28) und/oder das mindestens eine Zusatzteil (38, 40) zumindest bereichsweise im Querschnitt trapezförmig ausgestaltet sind.

10. Vorrichtung nach einem der voranstehenden Ansprüche, **dadurch gekennzeichnet, daß** das mindestens eine Koppelelement (66) aus einem Carbonfasermaterial gefertigt ist.

11. Vorrichtung nach einem der voranstehenden Ansprüche, **dadurch gekennzeichnet, daß** das mindestens eine Koppelelement (66) ein bewegbar am Hauptteil (28) oder am Zusatzteil (38, 40) gelagertes Verbindungselement (46) aufweist, das in eine Aufnahme des Zusatzteiles (38, 40) bzw. des Hauptteiles (28) einführbar ist.

12. Vorrichtung nach Anspruch 11, **dadurch gekennzeichnet, daß** das Verbindungselement als Querträger (46) ausgestaltet ist, der quer zur Körperlängsachse (36) des Patienten verschiebbar gelagert ist.

13. Vorrichtung nach Anspruch 11 oder 12, **dadurch gekennzeichnet, daß** das Verbindungselement (46) in einer am Zusatzteil (38, 40) festgelegten Führung (44) verschiebbar gelagert ist und in eine Aufnahme (50) des Hauptteiles einführbar ist.

14. Vorrichtung nach einem der voranstehenden Ansprüche, **dadurch gekennzeichnet, daß** die Vorrichtung (26) an ein Tischplattensegment (16) eines Patientenlagerungstisches (10) ankoppelbar ist.
